# EUROPÄISCHE PATENTANMELDUNG

(11) **EP 2 213 196 A1**
(43) Veröffentlichungstag der Anmeldung: **04.08.2010**
(21) Anmeldenummer: 09015152.3
(22) Anmeldetag: 07.12.2009
(51) Int. Cl.: A45D 29/05

(54) **Bearbeitungsinstrument**

(30) Priorität: 01.12.2008 DE 202008015821 U
(71) Anmelder: Busch & Co.KG, 51766 Engelskirchen (DE)
(72) Erfinder: Busch, Gert, 51766 Engelskirchen (DE)
(74) Vertreter: Maxton Langmaack & Partner

(57) **Zusammenfassung**

Die vorliegende Erfindung betrifft ein zum Behandeln einer Oberfläche von Köperextremitäten von Säugetieren, insbesondere von Nägeln oder Haut von Menschen, ausgelegtes, rotierendes Bearbeitungsinstrument (1), welches einen länglich verlaufenden Schaft (2) aufweist, der an einem Schaftende (3) einspannbar ist und an einem anderen Ende ein Arbeitsteil (4) mit einem die Oberfläche bearbeitenden, dabei Teile der Oberfläche abrasiv abnehmenden, rotationssymetrischen Arbeitsbereich (5) aufweist, der über einen Schaftdurchmesser hinausragt, wobei der Arbeitsbereich (5) an dem dem einzuspannenden Schaftende (3) entgegengesetzten Ende durch eine Abrundung (6) in kuppelähnlicher Gestalt (7) begrenzt ist, die ohne eine dem Arbeitsbereich (5) innewohnende Oberflächenbearbeitungsfunktion ausgestattet ist.

## Beschreibung

Die vorliegende Erfindung betrifft ein Bearbeitungsinstrument zum Behandeln einer Oberfläche von Körperextremitäten von Säugetieren, insbesondere von Nägeln oder Haut von Menschen. Hierzu wird ein rotierendes Bearbeitungsinstrument genutzt, welches einen länglich verlaufenden Schaft aufweist, der an einem Schaftende einspannbar ist und an einem anderen Ende ein Arbeitsteil mit einem die Oberfläche bearbeitenden, dabei Teile der Oberfläche abrasiv abnehmenden, rotationssymmetrischen Arbeitsbereich umfasst, der über einen Schaftdurchmesser hinausragt.

Bei einer Bearbeitung von Haut, Nägeln und Kunstnägeln beispielsweise in einer Fußpflegepraxis oder in einem Nagelstudio werden üblicherweise an die Morphologie der Körperextremität, d. h. des Fußes, der jeweiligen Nägel und daran gelegentlich auftretenden unterschiedlichen Deformationen angepasste Bearbeitungsinstrumente eingesetzt. Ein derartiges, insbesondere auch kühlbares rotierendes, einstückiges Bearbeitungsinstrument geht beispielsweise aus der DE 229 008 683 U1 hervor. Das dort hervorgehende Bearbeitungsinstrument ist insbesondere für die Fußpflege einsetzbar. Das rotierende Bearbeitungsinstrument ist mit Schleifmaterial versehen und im Übrigen aus Metall gefertigt. Die Außenfläche ist kappenförmig gestaltet, wobei als Schleifmittel eine Diamantkörnung aufgebracht ist.

Die DE 20 2005 018 372 U1 beschreibt ein Werkzeug für den Einsatz in der Fußpflege und der Kosmetik, bei dem einer Wärmeentwickung entgegengewirkt wird, indem der abrasive Werkzeugkopf sowie die zu bearbeitende Stelle durch einen Sprühnebel gekühlt wird. Zwar verringert diese Vorrichtung eine Verletzungsrisiko durch Verbrennung, jedoch besteht weiterhin das Risiko der Hautabschürfung.

Bei der Bearbeitung des Fußes oder der Hand, insbesondere von Nägeln wie auch Haut, kann es bei dem Gebrauch des Bearbeitungsinstruments, insbesondere bei unvorsichtigem Gebrauch von bekannten Fräs- oder Schleifinstrumenten zu einer Verletzungsgefahr durch den oberflächenwirksamen, insbesondere abrasiv, d. h. spanabhebend beziehungsweise schleifend wirkenden Bereich des Bearbeitungsinstruments an der Extremität kommen. So können Stellen der Oberfläche der Extremität bearbeitet werden, wo es nicht erwünscht ist, zum Beispiel am Nagelbett. Dies birgt insbesondere Risiken für Diabetiker wie auch für andere, mit bestimmten Leiden betroffene Personenkreise.

Aufgabe der vorliegenden Erfindung ist es, ein Bearbeitungsinstrument zur Verfügung zu stellen, das eine Bearbeitung von Extremitäten und ihren Oberflächen, insbesondere im Bereich der Fußpflegepraxis und Nagelstudios, deutlich im Risiko minimiert.

Diese Aufgabe wird mit einem Bearbeitungsinstrument mit den Merkmalen des Anspruches 1 sowie mit einem Vorprodukt mit den Merkmalen des Anspruches 13 gelöst. Weitere vorteilhafte Ausgestaltungen und Weiterbildungen sind in den jeweiligen Unteransprüchen angegeben. Die einzelnen Merkmale der beschriebenen Ausgestaltungen sind jedoch nicht auf diese beschränkt, sondern können mit weiteren Merkmalen aus den Ansprüchen oder der Beschreibung zu weiteren Ausgestaltungen verknüpft werden.

Zum Behandeln einer Oberfläche von Körperextremitäten von Säugetieren, insbesondere von Nägeln oder Haut von Menschen, wird ein dafür ausgelegtes, rotierendes Bearbeitungsinstrument vorgeschlagen, welches einen länglich verlaufenden Schaft aufweist, der an einem Schaftende einspannbar ist und an einem anderen Ende ein Arbeitsteil mit einem die Oberfläche bearbeitenden, dabei Teile der Oberfläche abrasiv abnehmenden, rotationssymmetrischen Arbeitsbereich aufweist, der über einen Schaftdurchmesser hinausragt, wobei der Arbeitsbereich an dem dem einzuspannenden Schaftende entgegengesetzten Ende durch eine Abrundung in kuppelähnlicher Gestalt begrenzt ist, die ohne eine dem Arbeitsbereich innewohnende Oberflächenbearbeitungsfunktion ausgestattet ist.

In einer Ausgestaltung weist das Arbeitstteil einen Durchmesser von 3 Millimeter bis 10 Millimeter auf , bevorzugt von 5 Millimeter bis 8 Millimeter, besonders bevorzugt etwa 6 Millimeter. Das Bearbeitungsinstrument ist gemäß einer weiteren Ausgestaltung derart ausgestaltet, dass der Schaft in ein elektrisches Instrument vorzugsweise zur Nagel und Hautbearbeitung eingespannt werden kann.

Es hat sich als vorteilhaft herausgestellt, wenn der kuppelähnliche Bereich zum einen eine Abrundung aufweist. Hierdurch wird eine Verletzungsgefahr, wie sie bei bisherigen Bearbeitungsinstrumenten insbesondere durch vorgesehene Abkantungen gegeben sein können, vermieden. Da darüber hinaus es sich als besonders vorteilhaft herausgestellt hat, in diesen Bereich der kuppelähnlichen Gestalt eine dem Arbeitsbereich innewohnende Oberflächenbearbeitungsfunktion wegzulassen und damit überhaupt nicht vorzusehen, kann das Bearbeitungsinstrument schon an die Oberfläche der Extremitäten angesetzt werden, ohne aber die Oberfläche selbst abrasiv zu bearbeiten. Wie oben schon dargelegt, ist unter "abrasiv" gemäß der hier vorgeschlagenen technischen Lehre zu verstehen, dass durch die dem Arbeitsbereich innewohnende Oberflächenbearbeitungsfunktion Material von der Oberfläche abgenommen werden kann. Dieses kann beispielsweise auf schleifende, fräsende oder sonstige Weise erfolgen. Bevorzugt ist vorgesehen, dass die kuppelähnliche Gestalt glatt ist, bei Verwendung eines Metalls vorzugsweise poliert. Dieses erlaubt beispielsweise, bei Anwendung des Bearbeitungsinstrumentes dieses zuerst auf der Oberfläche aufzusetzen und sich sodann an die Stelle herantasten zu können, bei der eine Oberfläche der Körperextremität tatsächlich zu bearbeiten ist. Durch ein Verschwenken des Bearbeitungsinstrumentes und/oder durch ein Tieferverfahren des Bearbeitungsinstrumentes tritt dann erst der Arbeitsbereich in Kontakt mit der Oberfläche, und ein abrasiver Materialabtrag an der Oberfläche vollzieht sich.

Gemäß einer Ausgestaltung kann vorgesehen sein, dass die kuppelähnliche Gestalt ein anderes Material aufweist, zumindest an der Oberfläche, als das Arbeitsteil. Beispielsweise kann die kuppelähnliche Gestalt eine Beschichtung aufweisen. Die kuppelähnliche Gestalt kann jedoch ebenfalls ein separates Element sein, dass das Arbeitsteil mitbildet. Hierzu kann beispielsweise die kuppelähnliche Gestalt aufgesetzt werden, beispielsweise durch Fügetechnik, Klebetechnik oder ähnliches. Eine weitere Ausgestaltung sieht vor, dass die kuppelähnliche Gestalt stoffschlüssiger Bestandteil des Arbeitsteils ist. Hierbei ist vorzugsweise vorgesehen, dass die kuppelähnliche Gestalt aus einem Vorprodukt herausgearbeitet wird, wenn das Arbeitsteil aus dem Vorprodukt hergestellt wird. Wird beispielsweise das Bearbeitungsinstrument mittels eines spanabhebenden Verfahrens hergestellt, beispielsweise mittels eines Drehvorganges, kann ein dabei verwendeter Drehmeißel die kuppelähnliche Gestalt so hervorbringen, dass gleichzeitig die Oberfläche in diesem Bereich glatt ist und insbesondere ein zusätzliches Polieren entfallen kann. Wird beispielsweise das Bearbeitungsinstrument mittels Feinguss hergestellt, kann die entsprechende Gussform eine derartige Oberflächengüte aufweisen, dass eine Nachbearbeitung der kuppelähnlichen Gestalt ebenfalls vorzugsweise entfallen kann.

Die kuppelähnliche Gestalt kann des Weiteren auch eine Beschichtung aufweisen. Diese Beschichtung kann beispielsweise eine Eigenschaft aufweisen, zum Beispiel nicht elektrisch leitfähig sein, elastisch sein, eine höhere Oberflächenglattheit aufzuweisen als ein anderes Material des Arbeitsteils, oder sonstiges. Die Beschichtung ist vorzugsweise temperaturfest und in der Lage, Temperaturen von zumindest 134° C und mehr unbeschadet auszuhalten. Diese Materialeigenschaft kann im Übrigen auch das separate Elemente aufweisen, das als kuppelähnliche Gestalt das Arbeitsteil mitbildet. Wird beispielsweise eine Beschichtung in Form eines Überzuges verwendet, kann der Überzug von sich aus eine Oberflächenglattheit zur Verfügung stellen, die ansonsten nur aufgrund einer Bearbeitung des Arbeitsteils im Bereich der kuppelähnlichen Gestalt ermöglicht werden könnte. Auch kann vorgesehen sein, dass die Beschichtung schon bei einem Vorprodukt Verwendung findet. Eine Ausgestaltung sieht beispielsweise vor, dass ein Vorprodukt zur Herstellung des Arbeitsteils des Bearbeitungsinstruments eine Beschichtung auf der kuppelähnlichen Gestalt aufweist, die verhindert, dass bei Auftragen insbesondere eines Schleifmaterials oder sonstigem abrasiven Materials für den Arbeitsbereich ein derartiges Anordnen des Verschleifmaterials auch auf der kuppelähnlichen Gestalt verhindert wird. Wird beispielsweise mittels eines speziellen Beschichtungsverfahrens das Schleifmaterial im späteren Arbeitsbereich des Arbeitsteils aufgetragen, kann durch eine Schutzbeschichtung vermieden werden, dass das Schleifmaterial im Bereich der kuppelähnlichen Gestalt anhaftet. Die Oberfläche in diesem Bereich bleibt damit von der Oberflächenbearbeitungsfunktion des Arbeitsbereiches frei. Gemäß einer weiteren Ausgestaltung ist vorgesehen, dass eine ein Anbringen eines Schleifmaterials verhindernde Schutzbeschichtung auch wieder entfernbar ist. Dieses kann beispielsweise über chemische Prozesse erfolgen. Beispielsweise kann ein galvanisches Auftragsverfahren genutzt werden, um das Schleifmaterial aufzutragen. Durch die Schutzbeschichtung wird eine elektrische Leitung jedoch verhindert, so dass sich an dem Ort der Schutzbeschichtung keinerlei Schleifmaterial anordnen kann. Verschiedene Materialien zur Nutzung für die Herstellung des Bearbeitungsinstrumentes wie auch bezüglich des Schleifmaterials sowie verschiedener Auftragstechniken und Eigenschaften hinsichtlich Sterilisation und/Desinfektion gehen beispielsweise aus der DE 20 2007 017 619 U1 hervor, auf die im Rahmen dieser Offenbarung vollständig verwiesen wird. Gleiches gilt auch für die dort angesprochene Verwendung von Matrixmaterialien für den Arbeitsbereich.

Die kuppelähnliche Gestalt ist vorzugsweise rotationssymmetrisch gestaltet. Zum einen vereinfacht dieses eine Herstellung. Zum anderen weist dadurch das Bearbeitungsinstrument bei Heranführung zuerst der kuppelähnlichen Gestalt an eine Oberfläche der Körperextremität immer zu jedem Zeitpunkt die gleiche Kontaktfläche auf, insbesondere sofern die kuppelähnliche Gestalt eine Vollkuppel ist, die entweder über Einbuchtungen, Einkerbungen oder andere von der kuppelähnlichen Gestalt als Vorgabe abweichende, die kuppelähnliche Gestalt als übergeordnete Geometrie weiterhin jedoch erkennen lassende Gestaltung aufweist. Eine weitere Ausgestaltung kann derartiges jedoch aufweisen. Dieses kann beispielsweise dadurch erzielt werden, dass ausgehend vom Arbeitsbereich ein beispielsweise halbkugelförmiges Zulaufen der kuppelähnlichen Gestalt erfolgt, wobei jedoch entlang des Umfangs betrachtet die Oberfläche mit Einbuchtungen versehen ist, wie sie analog beispielsweise bei einer Zitruspresse in ähnlicher Weise bekannt sind. Durch eine derartige Gestaltung wird der Nutzer des Bearbeitungsinstruments bei Heranführen desselben an die Oberfläche sofort darüber informiert, dass ein Kontakt hergestellt ist zwischen der zu bearbeitenden Oberfläche und dem Bearbeitungsinstrument. Durch eine beispielsweise leichte Unruhe aufgrund einer unregelmäßigen Gestaltung der kuppelähnlichen Gestalt wird dem Nutzer des Bearbeitungsinstruments sofort der Kontakt vermittelt und dadurch sichergestellt, dass gleichzeitig auch ein Unterschied eintritt in dem Moment, in dem der Arbeitsbereich mit der zu bearbeitenden Oberfläche in Kontakt tritt oder nur die kuppelähnliche Gestalt in Kontakt mit der zu bearbeitenden Oberfläche ist. Beispielsweise kann auch vorgesehen sein, dass der Arbeitsbereich derartige Erhebungen oder Ausnehmungen aufweist, die bei Kontakt mit der zu bearbeitenden Oberfläche dem Nutzer des Bearbeitungsinstruments ebenfalls ein entsprechendes Gefühl vermittelt, so dass der Nutzer auch hierüber tatsächlich eine Information erhält, dass der Arbeitsbereich die Oberfläche berührt.

Gemäß einer weiteren Ausgestaltung ist vorgesehen, dass das Arbeitsteil zumindest in Teilen austauschbar ist. Hierzu kann beispielsweise das gesamte Arbeitsteil vom Schaft getrennt werden. Es besteht jedoch ebenfalls die Möglichkeit, dass beispielsweise die kuppelähnliche Gestalt des Arbeitsteiles vom Rest trennbar ist beziehungsweise der Arbeitsbereich vom Bearbeitungsinstrument ausgetauscht werden kann. Hierzu ist beispielsweise vorgesehen, dass der Arbeitsbereich am Arbeitsteil derart angeordnet ist, dass der Arbeitsbereich lösbar ist. Dieses kann beispielsweise gewaltfrei erfolgen, indem beispielsweise eine Verklebung gelöst wird, worüber beispielsweise ein Schleifmaterial vom Arbeitsteil abgelöst werden kann. Auch andere chemische Verfahren, thermische Verfahren oder sonstige mechanische Verfahren zur Trennung des Arbeitsbereich, insbesondere von Schleifmaterial am Bearbeitungsinstrument können eingesetzt werden.

Gemäß einer weiteren Ausgestaltung ist vorgesehen, dass eine Oberfläche der kuppelähnlichen Gestalt in eine Oberfläche des Arbeitsbereichs stufenlos übergeht. Aufgrund des stufenlosen Überganges wird ein Verletzungsrisiko bei Nutzung des Bearbeitungsinstruments verhindert, da dadurch eine scharfe Kante gerade nicht gebildet wird, wie es bei anderen Bearbeitungsinstrumenten bisher der Fall gewesen ist. Zum anderen erlaubt dieses, dass die Bearbeitungsoberfläche des Arbeitsbereichs in die kuppelähnliche Gestalt stufenlos übergeht. Auf diese Weise kann ebenfalls eine Stufe vermieden werden, die eventuelle Verletzungsgefahren ansonsten bei Einsatz des Bearbeitungsinstrumentes aufweisen könnte. Vorzugsweise ist vorgesehen, dass in einer Ausnehmung im Arbeitsteil entlang der Erstreckung des Bearbeitungsinstruments der Arbeitsbereich angeordnet ist. Auf diese Weise kann die Ausnehmung beispielsweise mit Schleifmaterial gefüllt werden und somit eine gleichmäßige Anpassung der Bearbeitungsoberfläche des Arbeitsbereichs an eine Oberfläche der kuppelähnlichen Gestalt angepasst werden. Des Weiteren besteht durch die Möglichkeit der Anordnung einer Ausnehmung im Arbeitsteil die Möglichkeit, hierüber zu verhindern, dass ein unbeabsichtigter Kontakt des Arbeitsbereichs mit den zu bearbeitenden Oberflächen der Extremitäten erfolgt. Gemäß einer Ausgestaltung ist hierfür, aber auch unabhängig davon, vorgesehen, dass die kuppelähnliche Gestalt über den Arbeitsbereich hinausragt und damit ebenfalls einen gewissen Schutz bietet. Hierbei kann beispielsweise vorgesehen sein, dass der Durchmesser der kuppelähnlichen Gestalt zumindest in einem Abschnitt größer ist als ein Durchmesser des Arbeitsbereiches.

Der Arbeitsbereich weist zumindest in einem Abschnitt einen Fräsabschnitt auf. Hierzu können gleiche oder verschiedene Fräsgeometrien eingesetzt werden, um einen derartigen Fräsabschnitt zu erzeugen. Der Verlauf der Frässchneiden kann parallel zu der Schaftachse wie auch geneigt dazu erfolgen. Beispielsweise können die Fräskanten geradlinig verlaufen, sie können jedoch auch einen gebogenen Verlauf zumindest über einen Abschnitt des Fräsabschnittes aufweisen.

Eine weitere Ausgestaltung sieht vor, dass der Arbeitsbereich zumindest einen Schleifbereich aufweist. Auch besteht die Möglichkeit, dass der Arbeitsbereich nur einen Fräsabschnitt oder aber nur einen Schleifbereich aufweist. Ebenfalls ist die Möglichkeit gegeben, dass der Arbeitsbereich einen Fräsabschnitt und einen Schleifbereich aufweist. Des Weiteren ist die Möglichkeit gegeben, dass der Schleifbereich eine unterschiedliche Körnung aufweist, wobei vorzugsweise verschiedene Bereiche mit unterschiedlicher Körnung vorhanden sind. So kann gemäß einer Ausgestaltung vorgesehen sein, dass eine feinere Körnung näher zur kuppelähnlichen Gestalt im Arbeitsteil angeordnet ist als eine gröbere Körnung. Von einer feineren zu einer gröberen Körnung kann gemäß einer Ausgestaltung der Arbeitsbereich graduelle Unterschiede aufweisen, die abschnittsweise jeweils eine Körnung mit durchschnittlich gleichem Körnungsdurchmesser aufweist. Gemäß einer anderen Ausgestaltung wird vorgesehen, dass eine kontinuierliche Änderung des durchschnittlichen Körnungsdurchmessers über die Länge des Arbeitsbereiches entlang der Schaftachse vorgesehen ist. Wiederum eine andere Ausgestaltung weist einen Arbeitsbereich auf, der einen oder mehr verschiedene Abschnitte unterschiedlicher durchschnittlicher Körnung und einen Abschnitt mit kontinuierlich sich änderndem Körnungsdurchmesser hat.

Vorzugsweise ragt die kuppelähnlich Gestalt über zumindest einen Teil des Arbeitsbereichtes hinaus. In einer weiteren Ausgestaltung ist vorgesehen, dass das Bearbeitungsinstrument als Vorprodukt aus dem Vollen hergestellt und anschließend der Arbeitsbereich erstellt wird.

Ein weiterer Gedanke der Erfindung umfasst ein Vorprodukt zur Herstellung eines Arbeitsteils eines Bearbeitungsinstruments, wobei eine kuppelförmige Gestalt an einem Ende des Arbeitsteils mit einer vorzugsweise entfernbaren Beschichtung versehen ist, die eine Anordung eines Schleifmaterials dort verhindert.

Ein weiterer Gedanke der Erfindung umfasst ein Verfahren zur Herstellung eines Bearbeitungsinstruments, vorzugsweise eines oben beschriebenen Bearbeitungsinstruments, wobei das Bearbeitungsinstrument aus einem Vollen hergestellt und anschließend der Arbeitsbereich erstellt wird. Eine kuppelähnlich Gestalt am Ende des Arbeitsteils wird mit einer vorzugsweise entfernbaren Beschichtung versehen, die eine Anordnung eines Schleifmaterials dort verhindert. In einer Ausgestaltung wird die Beschichtung nach einem Aufbringen eines Schleifmaterials entfernt. Ist der Arbeitsbereich aus einem metallischen Material, so ist in einer Ausgestaltung vorgesehen, dass insbesondere in einem letzten Arbeitsschritt die kuppelähnliche Gestalt poliert wird.

Weitere vorteilhafte Ausgestaltungen und Weiterbildungen sind aus den nachfolgenden Figuren zu entnehmen. Die aus den Figuren jeweils hervorgehenden Einzelheiten sind jedoch nicht auf die einzelne Ausgestaltung beschränkt. Vielmehr können ein oder mehrere dieser Merkmale mit anderen Merkmalen aus den Figuren wie auch aus der obigen Beschreibung zu Weiterbildungen verknüpft werden, die hier nicht in aller Ausführlichkeit näher dargelegt sind, und dennoch zum Offenbarungsgehalt der technischen Lehre gehören.

Es zeigen:
- Fig. 1: eine erste Ausgestaltung eines Bearbeitungsinstruments,
- Fig. 2: eine zweite Ausgestaltung eines Bearbeitungsinstruments,
- Fig. 3: eine dritte Ausgestaltung eines Bearbeitungsinstruments,
- Fig. 4: eine vierte Ausgestaltung eines Bearbeitungsinstruments,
- Fig. 5: eine fünfte Ausgestaltung eines Bearbeitungsinstruments und
- Fig. 6: eine sechste Ausgestaltung eines Bearbeitungsinstruments.

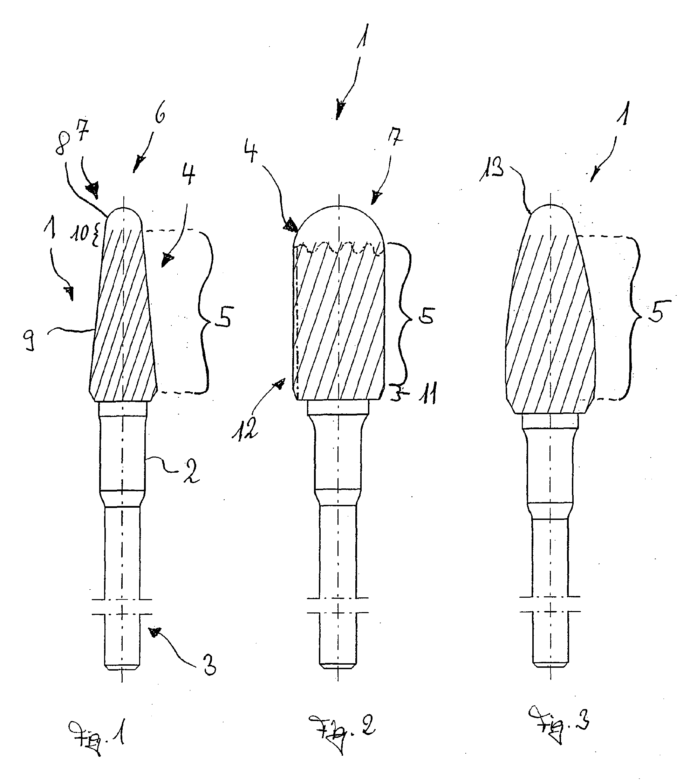

Fig. 1 zeigt eine erste Ausgestaltung eines Bearbeitungsinstruments 1. Mittels des Bearbeitungsinstruments 1 kann eine Oberfläche einer Körperextremität behandelt werden. Hierzu weist das Bearbeitungsinstrument 1 einen Schaft 2 auf, der beispielsweise mit gleichem oder wie hier stufenartigem Schaftverlauf versehen ist. Ein Schaftende 3 ist einspannbar. Hierzu kann der Schaft 2 an seinem Schaftende 3 entsprechende Schaftnuten oder sonstige Ausnehmungen aufweisen, die die Übertragung einer Rotationskraft ermöglichen. Es besteht ebenfalls die Möglichkeit, dass anstelle von Ausnehmungen auch Erhebungen am Schaftende 3 hierfür vorgesehen sind. Das Schaftende 3 kann darüber hinaus jedoch ebenfalls so wie dargestellt ausschließlich zylindrisch gestaltet sein, wobei eine Drehmomentübertragung durch Klemmung des Schaftendes 3 in einem entsprechend gestalteten Aufnahmewerkzeug erfolgt. Entgegengesetzt zum Schaftende 3 ist ein Arbeitsteil 4 angeordnet. Das Arbeitsteil 4 weist einen Arbeitsbereich 5 auf. Dieser kann beispielsweise durch vorgesehene Frässchneiden und/oder Schleifmaterial gebildet sein. Das Arbeitsteil 4 weist darüber hinaus an dem zum Schaftende 3 entgegengesetzten Ende eine Abrundung 6 in Form einer kuppelähnlichen Gestalt 7 auf. Vorzugsweise ist die kuppelähnliche Gestalt 7 so wie dargestellt glatt und eine Oberfläche 8 der kuppelähnlichen Gestalt geht stufenlos in eine Oberfläche 9 des Arbeitsbereiches 5 über. Ein derartiger Übergangsbereich 10 vermeidet beispielsweise, dass das Bearbeitungsinstrument bei Nutzung in der Tiefe und wieder Herausziehen sich versehentlich verkanten und damit stecken bleiben kann. Eine Gesamtkontur des Arbeitsteils 4 ist gemäß dieser ersten Ausgestaltung nicht nur rotationssymmetrisch, vielmehr verläuft sie von einem Ende zum anderen mit ansteigendem Durchmesser, der stetig zunimmt. Gemäß dieser Ausgestaltung weist das Arbeitsteil 4 eine im Wesentlichen konische Geometrie auf.

Im Folgenden werden gleichartige Elemente mit gleichen Bezugszeichen bezeichnet, ohne dass dieses jedoch beschränkend hinsichtlich des Schutzbereichs aufzufassen sei.

Fig. 2 zeigt eine zweite Ausgestaltung des Bearbeitungsinstruments 1. Auch hier ist ein Arbeitsbereich 5 am Arbeitsteil 4 vorgesehen. Die kuppelähnliche Gestalt 7 verläuft auch hier regelmäßig über in den Arbeitsbereich 5. Die kuppelähnliche Gestalt 7 kann gemäß einer weiteren Ausgestaltung, die allerdings nur gestrichelt dargestellt ist, einen unterschiedlichen Übergang in den Arbeitsbereich 5 aufweisen. Der Arbeitsbereich 5 ist gemäß der zweiten Ausgestaltung des Bearbeitungsinstrumentes 1 zylindrisch gestaltet. Auch hier ist vorgesehen, dass beispielsweise eine Frässchneide oder ein Schleifmaterial über den eigentlichen Arbeitsbereich 5 hinaus in einem Auslaufbereich 11 angeordnet sein kann. Der Auslaufbereich 11 ist vorzugsweise so gestaltet, dass dieser eine Anphasung, eine Abrundung oder zumindest eine Entgratung aufweist, so dass eine Verletzungsgefahr bei eventuellem Kontakt des Auslaufbereichs 11 mit der zu bearbeitenden Oberfläche vermieden wird. Des Weiteren ist schematisch mittels einer strichpunktierten Linie bei dem zweiten Ausführungsbeispiel des Bearbeitungsinstruments 1 eine mögliche Ausnehmung 12 im Arbeitsteil 5 dargestellt. In diese Ausnehmung kann beispielsweise ein Schleifmaterial oder ein sonstiges mit einer Oberflächenbearbeitungsfunktion versehenes Material angeordnet werden.

Fig. 3 zeigt eine weitere dritte Ausgestaltung des Bearbeitungsinstruments 1. Das dritte Bearbeitungsinstrument 1 ist beispielsweise vollständig einstückig hergestellt aus einem Stahlmaterial. Hierzu wird vorzugsweise das gesamte Bearbeitungsinstrument 1 gedreht, wobei bei einem derartigen Vorprodukt beispielsweise die kuppelähnliche Gestalt 7 mit einer Beschichtung 13 versehen werden kann. Diese Beschichtung 13 kann beispielsweise verhindern, dass ein Schleifmaterial auch auf diesen Bereich mit aufgetragen werden kann. Die Beschichtung kann auch zusätzliche Aufgaben erfüllen, beispielsweise eine Kennzeichnung ermöglichen. Weisen die Beschichtungen 13 unterschiedliche Farben auf, kann diesen jeweils eine Körnung des verwendeten Schleifmaterials zugeordnet sein. Dadurch kann verhindert werden, dass der Nutzer bei der Auswahl zwischen verschiedenen Bearbeitungsinstrumenten, die aufgereiht abgestellt in entsprechenden Ständern vorhanden sind, erst einmal nach entsprechenden Hinweisen zu dem jeweiligen Körnungsgrad beispielsweise am Schaft angeordnet suchen muss.

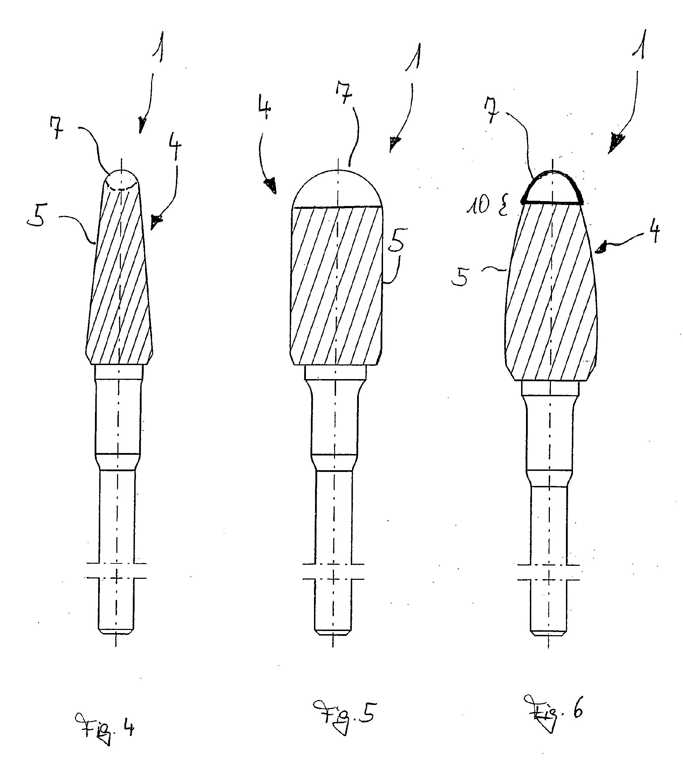

Fig. 4 zeigt eine vierte Ausgestaltung des Bearbeitungsinstruments 1. Die Formgestaltung stimmt mit derjenigen aus Fig. 1 überein. Allerdings ist bei der hier vorgesehenen Variante beispielhaft angegeben, dass die kuppelähnliche Gestalt 7 aus einem anderen Material ist als der übrige Bereich des Arbeitsteils 4. Dieses ist beispielhaft durch die gestrichelte Linie angedeutet, die den abgerundeten Bereich der kuppelähnlichen Gestalt von dem Arbeitsbereich 5 des Arbeitsteils 4 abtrennt. Die kuppelähnliche Gestalt 7 kann beispielsweise ein Kunststoffmaterial sein, insbesondere ein gummiartiger Kunststoff. Beispielsweise kann das Material elastische Eigenschaften aufweisen. Beispielsweise kann das Material der kuppelähnlichen Gestalt auf das ansonsten annähernd fertiggestellte Vorprodukt aufgebracht werden, beispielsweise in Form eines größeren flüssigen Tropfens des Materials, der sodann verfestigt. Hierbei kann beispielsweise eine formschlüssige Verbindung zwischen der kuppelähnlichen Gestalt 7 und dem übrigen Bereich des Arbeitsteils 4 geschaffen werden. Es kann jedoch auch ein vorgefertigtes Element mit einer Oberflächengestaltung von der kuppelähnlichen Gestalt 7 vorgefertigt und sodann mit dem Arbeitsteil 4 verbunden werden.

Fig. 5 zeigt eine fünfte Ausgestaltung des Bearbeitungsinstruments 1. Die kuppelähnliche Gestalt 7 ist hierbei in Form einer Kugelhalbschale auf den Arbeitsbereich 5 angrenzend mit dem übrigen Arbeitsteil 4 verbunden. Die kuppelähnliche Gestalt 7 ist vorzugsweise ebenfalls aus einem anderen Material gestaltet als der übrige Bereich des Arbeitsteils 4.

Fig. 6 zeigt eine sechste Ausgestaltung, die sich an diejenige anlehnt, die aus Fig. 3 schon hervorgeht. Es wird bei der sechsten Ausgestaltung des Bearbeitungsinstruments 1 jedoch beispielhaft gezeigt, dass die kuppelähnliche Gestalt 7 auch kappenförmig mit dem Arbeitsteil 4 verbunden sein kann, wobei hierbei der Durchmesser der kuppelähnlichen Gestalt 7 im Übergangsbereich 10 zum Arbeitsbereich 5 größer ist. Das bedeutet, die Oberfläche der kuppelähnlichen Gestalt 7 geht nicht direkt über in die Oberfläche des Arbeitsbereiches 5. Vielmehr ist hierbei eine Differenz zu überbrücken. Diese Differenz kann beispielsweise mittels einer entsprechend gestalteten Rundung so ausgeführt sein, dass Verletzungsgefahren dadurch ausgeschlossen sind. Eine derartige kappenförmige Gestalt kann einstückig stoffschlüssig mit dem übrigen Material des Bearbeitungsinstruments 1 verbunden sein. Es besteht jedoch ebenfalls die Möglichkeit, hierfür ein anderes Material zu verwenden, insbesondere dieses lösbar vorzusehen.

Das vorgeschlagene Bearbeitungsinstrument ist besonders bestimmt zur Nutzung in Fußpflege- und Nagelstudios.

## Patentansprüche

1. Zum Behandeln einer Oberfläche von Köperextremitäten von Säugetieren, insbesondere von Nägeln oder Haut von Menschen, ausgelegtes, rotierendes Bearbeitungsinstrument (1), welches einen länglich verlaufenden Schaft (2) aufweist, der an einem Schaftende (3) einspannbar ist und an einem anderen Ende ein Arbeitsteil (4) mit einem die Oberfläche bearbeitenden, dabei Teile der Oberfläche abrasiv abnehmenden, rotationssymetrischen Arbeitsbereich (5) aufweist, der über einen Schaftdurchmesser hinausragt, **dadurch gekennzeichnet, dass** der Arbeitsbereich (5) an dem dem einzuspannenden Schaftende (3) entgegengesetzten Ende durch eine Abrundung (6) in kuppelähnlicher Gestalt (7) begrenzt ist, die ohne eine dem Arbeitsbereich (5) innewohnende Oberflächenbearbeitungsfunktion ausgestattet ist.

2. Bearbeitungsinstrument (1) nach Anspruch 1, **dadurch gekennzeichnet, dass** die kuppelähnliche Gestalt (7) glatt ist, vorzugsweise poliert.

3. Bearbeitungsinstrument (1) nach Anspruch 1 oder 2, **dadurch gekennzeichnet, dass** die kuppelähnliche Gestalt (7) ein anderes Material zumindest an der Oberfläche aufweist als das Arbeitsteil, wobei die kuppelähnliche Gestalt (7) ein separates Element ist, dass das Arbeitsteil (4) mitbildet und wobei die kuppelähnliche Gestalt (7) stoffschlüssiger Bestandteil des Arbeitsteils ist.

4. Bearbeitungsinstrument (1) nach Anspruch 5, **dadurch gekennzeichnet, dass** das Arbeitsteil (4) zumindest im Wesentlichen vollständig aus Hartmetall oder im Wesentlichen vollständig aus Stahl ist.

5. Bearbeitungsinstrument (1) nach einem der vorhergehenden Ansprüche, **dadurch gekennzeichnet, dass** die kuppelähnliche Gestalt (7) rotationssymetrisch ist.

6. Bearbeitungsinstrument (1) nach einem der vorhergehenden Ansprüche, **dadurch gekennzeichnet, dass** das Arbeitsteil (4) zumindest in Teilen austauschbar ist.

7. Bearbeitungsinstrument (1) nach einem der vorhergehenden Ansprüche, **dadurch gekennzeichnet, dass** eine Oberfläche (8) der kuppelähnlichen Gestalt (7) in eine Oberfläche (9) des Arbeitsbereichs stufenlos übergeht.

8. Bearbeitungsinstrument (1) nach einem der vorhergehenden Ansprüche, **dadurch gekennzeichnet, dass** eine Bearbeitungsoberfläche des Arbeitsbereichs in die kuppelähnliche Gestalt (7) stufenlos übergeht, wobei in einer Ausnehmung (12) im Arbeitsteil (4) entlang der Erstreckung des Bearbeitungsinstruments (1) der Arbeitsbereich (5) angeordnet ist.

9. Bearbeitungsinstrument (1) nach einem der vorhergehenden Ansprüche, **dadurch gekennzeichnet, dass** der Arbeitsbereich (5) zumindest einen Fräsabschnitt aufweist.

10. Bearbeitungsinstrument (1) nach einem der vorhergehenden Ansprüche, **dadurch gekennzeichnet, dass** der Arbeitsbereich (5) zumindest einen Schleifbereich aufweist, wobei der Arbeitsbereich (5) eine Schleifmittel-Beschichtung aufweist.

11. Bearbeitungsinstrument (1) nach einem der vorhergehenden Ansprüche, **dadurch gekennzeichnet, dass** das Arbeitsteil (4) aus Stahl ist und der Arbeitsbereich (5) durch eine Beschichtung mit einem Schleifmittel gebildet ist.

12. Bearbeitungsinstrument (1) nach einem der vorhergehenden Ansprüche, **dadurch gekennzeichnet, dass** die kuppelähnliche Gestalt (7) über zumindest einem Teil des Arbeitsbereichs hinausragt und das Bearbeitungsinstrument (1) als Vorprodukt aus dem Vollen hergestellt und anschließend der Arbeitsbereich (5) erstellt worden ist.

13. Vorprodukt zur Herstellung eines Arbeitsteils eines Bearbeitungsinstruments (1) nach einem der vorherigen Ansprüche, **dadurch gekennzeichnet, dass** eine kuppelähnliche Gestalt (7) an einem Ende des Arbeitsteils (4) mit einer vorzugsweise entfernbaren Beschichtung (13) versehen ist, die eine Anordnung eines Schleifmaterials dort verhindert.
